# FASCICULE DE BREVET EUROPEEN

(11) **EP 1 981 530 B1**
(45) Date de publication et mention de la délivrance du brevet: **08.07.2009**
(21) Numéro de dépôt: 07730852.6
(22) Date de dépôt: 24.01.2007
(51) Int. Cl.: A61K 38/48, A61K 8/64, A61P 21/00

(54) **COMPOSITION COMPRENANT PLUSIEURS TOXINES BOTULIQUES**
ZUSAMMENSETZUNG MIT MEHREREN BOTULINUMTOXINEN
COMPOSITION CONTAINING SEVERAL BOTULIC TOXINS

(30) Priorité: 27.01.2006 FR 0600732
(43) Date de publication de la demande: 22.10.2008
(73) Titulaire: IPSEN PHARMA, 92100 Boulogne-Billancourt (FR)
(72) Inventeur: BERRUET, Laure, 92370 Chaville (FR)
(74) Mandataire: Bourgouin, André
(86) Numéro de dépôt international: PCT/FR2007/000134
(87) Numéro de publication internationale: WO 2007/085728

(56) Documents cités:
- WO-A-00/55208
- WO-A-01/58472
- WO-A-94/28923
- US-A1- 2005 287 175
- DINEEN S S ET AL: "Nucleotide sequence and transcriptional analysis of the type A2 neurotoxin gene cluster in Clostridium botulinum" FEMS MICROBIOLOGY LETTERS, AMSTERDAM, NL, vol. 235, no. 1, 1 juin 2004 (2004-06-01), pages 9-16, XP002350000 ISSN: 0378-1097

## Description

La présente invention a pour objet une composition comprenant plusieurs toxines botuliques de type A.

La toxine botulique, en particulier la toxine botulique de type A1 (Dysport^{®} commercialisé par Ipsen ou Botox^{®} commercialisé par Allergan), est utilisée depuis les années 80 chez l'homme pour le traitement de maladies / désordres divers et variés. Parmi les maladies / désordres pouvant être traités par la toxine botulique, on peut citer entre autres des désordres musculaires (par exemple le blépharospasme, la spasticité de l'adulte ou de l'enfant ou encore le torticolis), la migraine, la, douleur d'origine musculaire, la douleur neuropathique, le diabète, l'hyperhydrose (ou transpiration excessive), l'hypersalivation ou même les rides.

Les applications des toxines botuliques connues à ce jour ont trait à son administration classique, intra-musculaire telle que décrite dans les traitements mentionnés. L'injection dans les muscles provoque leur paralysie transitoire, c'est à dire bloque les contractions musculaires sur une certaine période de temps.

Or les compositions de toxines botuliques utilisées à ce jour présentent une durée d'action dans le temps qui est limitée.

Afin de répondre aux exigences des industriels il est devenu nécessaire de trouver un moyen pour augmenter la durée d'action de la toxine botulique sans altérer son activité biologique. °

Aussi le problème que se propose de résoudre l'invention est de fournir une nouvelle composition de toxines botuliques ayant une durée d'action prolongée.

De manière inattendue, les inventeurs ont mis en évidence qu'il est possible de combiner plusieurs toxines botuliques.

Dans ce but la présente invention propose une composition comprenant au moins :
- une neurotoxine botulique de type A1, et
- une neurotoxine botulique de type A dont la séquence en acides aminés présente au moins 5 % de différence avec la séquence en acides aminés de la neurotoxine botulique de type A1.

L'invention offre des avantages déterminants, en particulier la présente composition présente une durée de la paralysie musculaire qui est améliorée lorsque ladite composition est injectée dans un muscle lisse ou squelettique. La durée de la paralysie musculaire peut atteindre jusqu'à 10 mois dans certains cas, et plus généralement varie entre 2 à 8 mois.

L'invention offre comme autre avantage que les compositions selon l'invention peuvent être utilisées à des doses beaucoup plus faibles comparées aux doses des toxines botuliques actuellement disponibles dans le commerce. En d'autres termes, pour obtenir la même relaxation des muscles, on injecte une quantité inférieure de toxine botulique. Pour les mêmes indications thérapeutiques, une réduction comprise entre 10 à 70 %, de préférence entre 25 à 50 %, des doses d'administration a pu être observée (comparaison entre unités de toxines injectées pour obtenir le même effet biologique).

Un autre avantage des compositions selon l'invention est qu'elles provoquent peu d'effets secondaires, et notamment beaucoup moins d'effets secondaires que les compositions connues à ce jour de toxine botulique. En particulier la possibilité d'utiliser de faibles doses de composition selon l'invention permet avantageusement de réduire les effets secondaires. Parmi les effets secondaires qui sont évités, on peut citer ceux lié à l'immunogénicité de la protéine elle-même, ainsi que la dysphagie, la ptose ou la faiblesse musculaire générale, cette liste n'étant pas exhaustive.

De plus, les compositions selon l'invention présentent comme autre avantage une rapidité d'action améliorée lorsque ladite composition est injectée dans un muscle lisse ou squelettique. L'effet biologique ou clinique peut apparaître 12 heures après l'injection, et plus généralement dans un délai compris entre 14 à 24 heures après l'injection.

Avantageusement, les compositions selon l'invention procurent un effet myorelaxant très homogène.

Enfin l'invention a pour avantage de pouvoir être mise en oeuvre dans toutes industries, notamment l'industrie pharmaceutique, vétérinaire, cosmétique, ainsi que dans les domaines de l'hygiène au quotidien ou de l'hygiène corporelle.

D'autres avantages et caractéristiques de l'invention apparaîtront clairement à la lecture de la description et des exemples donnés à titre purement illustratifs et non limitatifs qui vont suivre.

La présente invention a pour objet une composition comprenant au moins :
- une neurotoxine botulique de type A1, et
- une neurotoxine botulique de type A dont la séquence en acides aminés présente au moins 5 % de différence avec la séquence en acides aminés de la neurotoxine botulique de type A1.

Par l'expression neurotoxine botulique, on entend une toxine botulique qui est soit une protéine libre (i.e. libre de toute protéine la complexant), soit un complexe protéique, ledit complexe protéique pouvant comprendre par exemple de l'hémagglutinine (protéine HA) associée à de la toxine botulique, ou soit un fragment protéique.

Par l'expression toxine botulique, on entend une molécule possédant l'activité biologique de la toxine botulique, qui peut être soit une protéine, soit un polypeptide, soit un peptide, soit une protéine de fusion, soit une protéine tronquée, soit une protéine chimérique, soit une protéine mutée ou une protéine recombinante.

Par l'expression activité biologique de la toxine, on entend au sens de la présente invention soit une paralysie musculaire soit une inhibition de l'exocytose, en particulier de l'exocytose de l'acétycholine ou d'un autre neurotransmetteur.

Par protéine, polypeptide ou peptide on entend au sens de la présente invention, un polymère d'acides aminés, naturels ou non, lévogyre ou non, dextrogyre ou non.

Par protéine chimérique, on entend au sens de la présente invention une protéine obtenue après association de différents types de molécules, par exemple après association de lipides, de glycolipides, de peptides, de polypeptides, de protéines, de glycoprotéines, de carbohydrates, de polysaccharides, d'acides nucléiques, de polyéthylène glycol, etc.

La neurotoxine botulique, pure ou quasiment pure, peut être obtenue à partir d'un complexe protéique comprenant de la toxine botulique par exemple selon la méthode décrite dans Current topics in Microbiology and Immunology (1995), 195, p. 151-154. Une neurotoxine botulique, pure ou quasiment pure, peut être obtenue par exemple, par purification d'un milieu de fermentation ou bouillon de culture contenant une souche de *Clostridium Botulinum,* et enrichi par exemple en viande ou en nourriture protéinée.

Le premier constituant essentiel de la composition est constitué par une neurotoxine botulique de type A1.

La neurotoxine botulique de type A1 correspond en fait à la toxine botulique classique qui est communément appelée toxine botulique de type A, sans distinction du sous-type. La neurotoxine botulique de type A1 est commercialisée sous le nom de DYSPORT^{®} ou BOTOX^{®}.

Selon l'invention, la neurotoxine botulique de type A1 peut correspondre soit à un complexe de toxine botulique A1 et d'hémagglutinine, soit à la toxine botulique de type A1 libre de toutes protéines complexantes.

Le deuxième constituant essentiel de la composition est constitué par une neurotoxine botulique de type A dont la séquence en acides aminés présente au moins 5 % de différence avec la séquence en acides aminés de la neurotoxine botulique de type A1. Pour une lecture plus aisée dans la suite de l'exposé, cette neurotoxine est appelée neurotoxine botulique de type Ax.

Par l'expression "une neurotoxine botulique de type A dont la séquence en acides aminés présente au moins λ % de différence avec la séquence en acides aminés de la neurotoxine botulique de type A1", on entend que ces séquences en acides aminés (chaîne lourde et chaîne légère) correspondent aux séquences de la toxine botulique libre de toutes protéines complexantes. Par exemple, 10 % de différence, signifie que 10 acides aminés sont différents parmi 100 acides aminés. Il est entendu que différent peut signifier manquant, dans l'exemple 10% de différence peut s'entendre par 10 acides aminés manquants parmi 100 acides aminés.

Plus particulièrement la composition selon l'invention comprend au moins une neurotoxine botulique de type A dont la séquence en acides aminés présente au moins 8 % de différence avec la séquence en acides aminés de la neurotoxine botulique de type A1.

De préférence la composition selon l'invention comprend au moins une neurotoxine botulique de type A dont la séquence en acides aminés présente au moins 10 % de différence avec la séquence en acides aminés de la neurotoxine botulique de type A1.

Encore plus préférentiellement la composition selon l'invention comprend au moins une neurotoxine botulique de type A dont la séquence en acides aminés présente au moins 12 % de différence avec la séquence en acides aminés de la neurotoxine botulique de type A1.

Parmi les neurotoxines botuliques de type Ax, on peut citer la toxine botulique de type A2.

De préférence, la composition selon l'invention comprend au moins :
- une neurotoxine botulique de type A1, et
- une neurotoxine botulique de type A2.

La toxine botulique de type A2 a d'abord été isolée à partir de cas d'enfants atteints de botulisme vers 1990 (Sakaguchi et al., Int. J. Food Microbiol. (1990), 11, 231-242). Cette toxine est immunologiquement et biochimiquement différente de la toxine botulique de type A1.

La toxine botulique de type A2 peut être isolée à partir des souches suivantes : Kyoto-F, Chiba-H, Y-8036, 7103-H, 7105-H, KZ1828, NCTC2012 ou NCTC9837 (Cordoba et al., System. Appl. Microbiol. (1995), 18, 13-22; Franciosa et al., abstract presented at 40^{th} Interagency Botulism Research Coordinating Committee (IBRCC) Meeting, November 2003).

De préférence la composition selon l'invention comprend la toxine botulique de type A2 isolée à partir de la souche *Clostridium botulinum* référencée et accessible sous le numéro NCTC9837, auprès de la National Collection of Type Cultures - Central Public Health Laboratory - London - UK. La souche NCTC9837 est parfois appelée souche Mauritius 1955.

Encore plus préférentiellement, la composition selon l'invention comprend au moins :
- une neurotoxine botulique de type A1, et
- une neurotoxine botulique de type A2 isolée à partir de la souche *Clostridium botulinum* NCTC9837.

La toxine botulique de type A2 diffère de la toxine A1 par entre autre, sa séquence en acides aminés, son poids moléculaire, ses caractéristiques immunologiques et génétiques (Kubota et al., Biochem. Biophys. Res. Commun. (1996), 224 (3), 843-848).

Les différences biochimiques entre la toxine botulique de type A2 et celle de type A1 sont entre autre que la toxine botulique de type A1 contient la protéine NTNH (protéine non toxique et non-hémagglutinine) et au moins 3 hémagglutinines (HA17, HA34 et HA70), alors que la toxine botulique de type A2 peut contenir la protéine NTNH, sans hémagglutinine (Sakaguchi et al., Int. J. Food Microbiol. (1990), 11, 231-242).

La toxine botulique de type A2 possède une chaîne lourde d'environ 101 kDa alors que la toxine botulique de type A1 possède une chaîne lourde d'environ 93 kDa (Kozaki et al., Microbiol. Immunol. (1995), 39(10), 767-74).

La séquence en acides aminés des toxines botuliques de type A2 et A1 est significativement différente, plus particulièrement au niveau de la chaîne lourde. Par exemple dans la cas de la toxine botulique de type A2 issu de la souche Kyoto-F, 109 acides aminés parmi les 847 des acides aminés de la chaîne lourde sont différents entre les 2 toxines (13 % de différence) (Cordoba et al., System. Appl. Microbiol. (1995), 18, 13-22).

De manière générale, les chaînes lourdes des toxines botuliques de types A1 isolées diffèrent entre elles par moins de 2 % de différence. La chaîne lourde de la toxine botulique participe aux activités biologiques principales de la molécule, incluant la liaison au récepteur sur les cellules cibles et le transport intracellulaire (Zhang et al., Gene (2003), 315, 21-32).

Il existe des différences immunologiques entre la toxine botulique de type A2 et celle de type A1. En effet des anticorps dirigés contre la toxine botulique de type A1 ne reconnaissent pas la toxine botulique de type A2, et vice et versa. (Sakaguchi et al., Int. J. Food Microbiol. (1990), 11, 231-242; Kozaki et al., Microbiol. Immunol. (1995), 39(10), 767-74).

La présente composition selon l'invention peut en outre comprendre au moins un agent analgésique.

Parmi les agents analgésiques convenant selon la présente invention on peut citer un agent analgésique choisi parmi les inhibiteurs des canaux sodiques.

La présente composition selon l'invention peut en outre comprendre un mélange d'agents analgésiques cités ci-dessous.

Par "inhibiteurs des canaux sodiques", on entend par exemple les composés suivants (éventuellement sous forme de sels pharmaceutiquement acceptables) :
- la lidocaïne ;
- la tétracaïne ;
- la bupivacaïne ;
- la procaïne ;
- la mépivacaïne ; ou
- la dibucaïne ;

La présente composition selon l'invention peut en outre comprendre au moins un polysaccharide ou un mélange de plusieurs polysaccharides.

Par polysaccharide, on entend au sens de la présente invention, un polymère comprenant au moins 2 monomères, les monomères étant des saccharides. Cette définition inclut les disaccharides.

Dans le cadre de l'invention, les polysaccharides peuvent être ioniques et/ou non ioniques.

De préférence, la composition comprend au moins un polysaccharide comprenant majoritairement des unités de glucose. Le terme majoritairement signifiant, que le glucose est majoritaire en nombre d'unités de monomère.

Comme exemple de polysaccharides convenant selon l'invention, on peut citer l'amidon, les dérivés d'amidon, l'hydroxyethyle amidon en particulier le 2-hydroxy-ethyl amidon.

Les polysaccharides convenant selon la présente invention peuvent être substitués, en particulier peuvent être substitués par des radicaux alkyle, alcoxy, ou encore par des radicaux alkyle eux-mêmes substitués par des fonctions alcools.

Selon une variante de l'invention, la quantité de polysaccharide comprise dans la composition selon l'invention est d'au moins 1 µg de polysaccharide pour 1 unité de toxine botulique. Selon le choix du polysaccharide, il est possible d'utiliser au moins 0,5 µg de polysaccharide pour 1 unité de toxine botulique.

La présente composition selon l'invention peut en outre comprendre au moins un tensio-actif ou un mélange de plusieurs tensio-actifs.

Par agent tensio-actif, on entend au sens de l'invention un agent émulsifiant ou un agent solubilisant.

Dans le cadre de l'invention les tensio-actifs mis en oeuvre peuvent être choisis parmi les tensio-actifs cationiques, anioniques ou non-ioniques.

De préférence la composition selon l'invention comprend au moins un tensio-actif choisi parmi les tensio-actifs non-ioniques du groupe des polysorbates.

Parmi le groupe des polysorbates, on peut citer le polysorbate 20, le polysorbate 21, le polysorbate 40, le polysorbate 60, le polysorbate 61, le potysorbate 65, le polysorbate 80, le polysorbate 81, le polysorbate 85, le polysorbate 120, le polysorbate 80 acetate.

Le tensio-actif préféré selon une variante de la composition selon l'invention est le polysorbate 80.

La présente composition selon l'invention peut en outre comprendre au moins un vasoconstricteur ou un mélange de plusieurs vasoconstricteurs.

Dans le cadre de l'invention les vasoconstricteurs mis en oeuvre peuvent être choisis par exemple parmi les vasoconstricteurs du type catecholamines ou noncatecholamines.

Parmi les vasoconstricteurs convenant selon la présente invention, on peut citer à titre d'exemple l'epinéphrine, la norepinéphrine, la lévonordefrine, les amphétamines, l'éphédrine, la phenyléphrine, l'endotheline.

La composition selon l'invention peut être sous forme d'un solide_{,} par exemple des poudres, des lyophilisats, des granules, des comprimés ou des liposomes. La composition selon l'invention sous forme solide peut être conservée par exemple à des températures inférieures à 4° C, ou inférieures à 0° C sans que son activité biologique soit altérée.

La composition selon l'invention peut se présenter sous forme d'une dispersion aqueuse, de particules de neurotoxine botulique dans un réseau gélifié.

La composition selon l'invention peut aussi se présenter sous forme liquide, par exemple, des solutions, des émulsions ou des suspensions.

L'administration de la composition selon l'invention se fera de préférence par injection comme par exemple par injection intramusculaire ou sous-cutanée.

Dans le cas des injections, la composition selon l'invention pourra être associée à un agent facilitant l'injection encore appelé véhicule d'injection ou vecteur d'injection.

Selon un mode de réalisation des compositions selon l'invention, le ratio des fractions d'unité de neurotoxine botulique de type A1 sur neurotoxine botulique de type Ax [unités A1 / unités Ax] peut être compris entre 1 / 99 et 99 / 1, avantageusement entre 5 / 95 et 95 / 5, ou entre 10 / 90 et 90 / 10.

Selon une variante de la composition selon l'invention, le ratio des fractions d'unité de neurotoxine botulique de type A1 sur neurotoxine botulique de type Ax est de 50/50.

Selon une autre variante de la composition selon l'invention, le ratio des fractions d'unité de neurotoxine botulique de type A1 sur neurotoxine botulique de type Ax est de 60 / 40.

Selon une autre variante de la composition selon l'invention, le ratio des fractions d'unité de neurotoxine botulique de type A1 sur neurotoxine botulique de type Ax est de 40 / 60.

La présente invention a également pour objet l'utilisation d'une composition selon l'invention, décrite ci-dessus, pour l'obtention d'un médicament destiné à traiter les troubles musculaires, les troubles neuromusculaires, les troubles neurologiques, les troubles orthopédiques, les troubles ophtalmologiques, les pathologies articulaires, les troubles endocriniens ou les troubles urologiques.

La présente invention a également pour objet l'utilisation d'une composition selon l'invention, décrite ci-dessus, pour l'obtention d'un médicament destiné à traiter le torticolis, le torticolis spasmodique, les troubles locaux de la spasticité des membres supérieurs et/ou inférieurs, la douleur, la douleur musculaire, la douleur due aux spasmes musculaires, la douleur myofasciale, les douleurs post-opératoires, les spasmes musculaires, le spasme hémifacial, le blépharospasme, le strabisme, l'asymétrie faciale, la dystonie musculaire, la paralysie cérébrale, les céphalées, la migraine, la fibromyalgie, la myalgie, l'hyperhydrose, la bromhydrose, la coxarthrose, l'arthrose de la hanche, l'épicondylite du coude, l'arthrite, l'arthrite rhumatoïde, les dyskinésies, l'achalasie, les dysfonctionnements des sphincters de Oddi, la pancréatite, la goutte, les fissures anales, la constipation, l'anismus, les spasmes de la valve pylorique, la vessie spastique, les spasmes de la vessie, l'incontinence urinaire, la rétention d'urine, l'hyperplasie prostatique, l'endométriose, le psoriasis, la rhinite, la rhinite allergique, l'obésité, l'hyperlacrimation, les fractures osseuses, les déchirures des tendons ou la pathologie de la coiffe des muscles rotateurs de l'épaule.

La présente invention a également pour objet l'utilisation d'une composition selon l'invention, décrite ci-dessus, pour l'obtention d'un produit cosmétique.

La présente invention a également pour objet l'utilisation d'une composition selon l'invention, décrite ci-dessus, pour traiter les lignes de froncement du visage, les rides du visage, les rides de la peau, les rides du contour de l'oeil, les sillons intersourcilliers, la calvitie, l'acné, la transpiration excessive ou la perte des cheveux.

La présente invention a également pour objet en tant que médicament, la composition selon l'invention décrite ci-dessus.

La présente invention a également pour objet une composition pharmaceutique comprenant la composition selon l'invention décrite ci-dessus.

La dose de la composition selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

La quantité thérapeutiquement efficace qu'il convient d'injecter varie également selon le nombre de muscles à traiter, ainsi que selon la masse de ces muscles.

De préférence, les doses de composition selon l'invention injectées seront comprises entre 5 et 2000 Unités de toxine botulique, plus préférentiellement de 10 à 1000 Unités de toxine botulique, encore plus préférentiellement de 25 à 500 Unités de toxine botulique.

Les exemples suivants illustrent l'invention sans en limiter la portée.

### EXEMPLES

La quantification des neurotoxines botuliques utilisées selon l'invention a été réalisée par la mesure d'une dose létale DL₅₀. Par DL₅₀ on entend au sens de la présente invention la dose létale ou encore dose semi létale d'une substance donnée. Il s'agit de la dose (ou quantité) qui conduit à la mort de 50 % des animaux testés d'un groupe. Une unité de toxine (U) correspond à la DL₅₀ chez la souris par voie intra péritonéale.

### Compostions utilisées dans les exemples :

Ci-dessous sont décrits les constituants essentiels des compositions utilisées dans les exemples. Les autres constituants présents n'ont pas été précisés.
Composition A :
   - 250 unités de neurotoxine botulique de type A1 (Dysport^{®})
   - 250 unités de neurotoxine botulique de type A2 isolée à partir de la souche *Clostridium botulinum* NCTC9837
Composition B :
   - 500 unités de neurotoxine botulique de type A1 (Dysport^{®})

### Exemple 1 : Blépharospasme (dose 120 unités) :

Un patient âgé d'une cinquantaine d'années souffrant de Blépharospasme, fermeture involontaire des yeux résultant de la contraction des muscles situés autour des yeux, a reçu plusieurs injections intramusculaires de la composition A selon l'invention, la dose totale de composition A injectée étant de 120 unités de neurotoxine botulique pour un volume total de 1 ml.

Le protocole d'injection s'est déroulé de la manière suivante : une aiguille stérile est introduite dans un flacon contenant la composition A afin d'y introduire 2,5 ml d'une solution injectable de chlorure de sodium à 0,9 %. On obtient ainsi une solution limpide contenant 200 unités/ml de neurotoxine botulique.

Un patient souffrant de blépharospasme bilatéral a reçu par injection 120 unités de neurotoxine botulique (composition A) par oeil (soit 0,6 ml, pour la dilution de 1 flacon contenant 500 unités de neurotoxine botulique (composition A) dans 2,5 ml). Les injections ont eu lieu, après nettoyage de la peau autour des yeux, par la voie sous-cutanée avec une seringue de 1 ml (aiguille gauge 23 ou 25).

Une dose de 0,1 ml (20 unités) de composition A a été injectée dans la partie interne et une dose de 0,2 ml (40 unités) de composition A dans la partie externe de la jonction entre les zones pré septales et orbitales des muscles orbiculaires supérieurs de chaque oeil. De la même manière une dose de 0,1 ml (20 unités) de composition A a été injectée dans la partie interne et une dose de 0,2 ml (40 unités) de composition A dans la partie externe de la jonction entre les zones présentables et orbitales des muscles orbiculaires inférieurs de chaque oeil. Lors de l'injection dans la paupière supérieure, l'aiguille doit être orientée de préférence de telle sorte que l'on évite le centre de la paupière, partie où s'insère le muscle releveur de la paupière. Lors des administrations ultérieures, la dose totale par oeil pourra être réduite à 80 unités (0,4 ml), c'est-à-dire 20 unités par site d'injection (soit 0,1 ml).

Chez ce patient la paralysie musculaire a débuté environ 12 heures après le début du traitement, et la paralysie a duré environ 20 à 21 semaines. Aucun ptôsis partiel ou complet n'a été observé.

Chez un autre patient du même âge et présentant des symptômes similaires, avec le même protocole mais en utilisant la composition B avec le même dosage, la paralysie musculaire a débuté environ 24 heures après le début du traitement, et la paralysie a duré environ 12 à 13 semaines.

### Exemple 2 : Blépharospasme (dose 60 unités):

Un patient âgé d'une cinquantaine d'années souffrant de Blépharospasme, fermeture involontaire des yeux résultant de la contraction des muscles situés autour des yeux, a reçu plusieurs injections intramusculaires de la composition A selon l'invention, la dose totale de composition A injectée étant de 60 unités de neurotoxine botulique pour un volume total de 1 ml.

Le protocole d'injection s'est déroulé de la manière suivante : une aiguille stérile est introduite dans un flacon contenant la composition A afin d'y introduire 2,5 ml d'une solution injectable de chlorure de sodium à 0,9 %. On obtient ainsi une solution limpide contenant 200 unités/ml de neurotoxine botulique.

Un patient souffrant de blépharospasme bilatéral a reçu par injection 60 unités de neurotoxine botulique (composition A) par oeil (soit 0,3 ml, pour la dilution de 1 flacon contenant 500 unités de neurotoxine botulique (composition A) dans 2,5 ml). Les injections ont eu lieu, après nettoyage de la peau autour des yeux, par la voie sous-cutanée.

Une dose de 0,05 ml (10 unités) de composition A a été injectée dans la partie interne et une dose de 0,1 ml (20 unités) de composition A dans la partie externe de la jonction entre les zones pré septales et orbitales des muscles orbiculaires supérieurs de chaque oeil. De la même manière, une dose de 0,05 ml (10 unités) de composition A a été injectée dans la partie interne et une dose de 0,1 ml (20 unités) de composition A dans la partie externe de la jonction entre les zones présentables et orbitales des muscles orbiculaires inférieurs de chaque oeil. Lors de l'injection dans la paupière supérieure, l'aiguille doit être orientée de préférence de telle sorte que l'on évite le centre de la paupière, partie où s'insère le muscle releveur de la paupière. Lors des administrations ultérieures, la dose totale par oeil pourra être réduite à 40 unités (0,2 ml), c'est-à-dire 10 unités par site d'injection (soit 0,05 ml).

Chez ce patient, la paralysie musculaire a débuté environ 24 heures après le début du traitement, et la paralysie a duré environ 12 à 13 semaines.

Les résultats sont donc similaires à ceux obtenus avec la composition B, pour une dose d'administration réduite de 50 % en nombre d'unité de toxine.

### Exemple 3 : Torticolis spasmodique

Une patiente âgée de 35 ans souffrant de torticolis spasmodique, a reçu plusieurs injections intramusculaires de la composition A selon l'invention, la dose totale de composition A injectée étant de 500 unités de neurotoxines botuliques pour un volume total de 1 ml.

Le protocole d'injection s'est déroulé de la manière suivante : une aiguille stérile est introduite dans un flacon contenant la composition A afin d'y introduire 1 ml d'une solution injectable de chlorure de sodium à 0, 9 %. On obtient ainsi une solution limpide contenant 500 unités /ml de neurotoxine botulique.

Une patiente souffrant de torticolis spasmodique a reçu par injection 500 unités de neurotoxine botulique (composition A) soit 1 ml, pour la dilution de 1 flacon de 500 unités dans 1 ml. La dose totale a été répartie entre les 2 ou 3 muscles cervicaux les plus atteints (sterno-cléido-mastoïdien, splénius, trapèze ou angulaire).

Chez cette patiente la paralysie musculaire a débuté environ 18 heures après le début du traitement. La paralysie a duré environ 20 à 21 semaines. Aucune dysphagie n'a été constatée.

Chez une autre patiente du même âge et présentant des symptômes similaires, avec le même protocole mais en utilisant la composition B avec le même dosage, la paralysie musculaire a débuté environ 48 à 60 heures après le début du traitement, et la paralysie a duré environ 12 à 13 semaines.

## Revendications

1. Composition comprenant au moins :
- une neurotoxine botulique de type A1, et
- une neurotoxine botulique de type A dont la séquence en acides aminés présente au moins 5 % de différence avec la séquence en acides aminés de la neurotoxine botulique de type A1.

2. Composition selon la revendication 1 **caractérisée en ce qu'**elle comprend au moins une neurotoxine botulique de type A dont la séquence en acides aminés présente au moins 10 % de différence avec la séquence en acides aminés de la neurotoxine botulique de type A1.

3. Composition selon l'une des revendications précédentes **caractérisée en ce qu'**elle comprend au moins
- une neurotoxine botulique de type A1, et
- une neurotoxine botulique de type A2.

4. Composition selon l'une des revendications précédentes **caractérisée en ce qu'**elle comprend au moins
- une neurotoxine botulique de type A1, et
- une neurotoxine botulique de type A2 isolée à partir de la souche *Clostridium botulinum* NCTC9837.

5. Composition selon l'une des revendications précédentes **caractérisée en ce qu'**elle comprend au moins un agent analgésique.

6. Composition selon l'une des revendications précédentes **caractérisée en ce qu'**elle comprend au moins un polysaccharide.

7. Composition selon l'une des revendications précédentes **caractérisée en ce qu'**elle comprend au moins un polysaccharide qui est le 2-hydroxy-ethyl amidon.

8. Composition selon l'une des revendications précédentes **caractérisée en ce qu'**elle comprend au moins un tensio-actif, choisi parmi les tensio-actifs cationiques, anioniques ou non-ioniques.

9. Composition selon l'une des revendications précédentes **caractérisée en ce qu'**elle comprend au moins un tensio-actif choisi parmi les tensio-actifs non-ioniques du groupe des polysorbates.

10. Composition selon l'une des revendications précédentes **caractérisée en ce qu'**elle comprend au moins un tensio-actif qui est le polysorbate 80.

11. Composition selon l'une des revendications précédentes **caractérisée en ce qu'**elle comprend au moins un vasoconstricteur.

12. Composition selon l'une des revendications 1 à 11, pour utilisation comme médicament.

13. Composition selon l'une des revendications 1 à 11 pour le traitement des troubles musculaires, des troubles neuromusculaires, des troubles neurologiques, des troubles orthopédiques, des troubles ophtalmologiques, des pathologies articulaires, des troubles endocriniens ou des troubles urologiques.

14. Composition selon l'une des revendications 1 à 11 pour le traitement du torticolis, du torticolis spasmodique, des troubles locaux de la spasticité des membres supérieurs et/ou inférieurs, de la douleur, de la douleur musculaire, de la douleur due aux spasmes musculaires, de la douleur myofasciale, des douleurs post-opératoires, des spasmes musculaires, du spasme hémifacial, du blépharospame, du strabisme, de l'asymétrie faciale, de la dystonie musculaire, de la paralysie cérébrale, des céphalées, de la migraine, de la fibromyalgie, de la myalgie, de l'hyperhydrose, de la bromhydrose, de la coxarthrose, de l'arthrose de la hanche, de l'épicondylite du coude, de l'arthrite, de l'arthrite rhumatoïde, des dyskinésies, de l'achalasie, des dysfonctionnements des sphincters de Oddi, de la pancréatite, de la goutte, des fissures anales, de la constipation, de l'anismus, des spasmes de la valve pylorique, de la vessie spastique, des spasmes de la vessie, de l'incontinence urinaire, de la rétention d'urine, de l'hyperplasie prostatique, de l'endométriose, du psoriasis, de la rhinite, de la rhinite allergique, de l'obésité, de l'hyperlacrimation, des fractures osseuses, des déchirures des tendons ou de la pathologie de la coiffe des muscles rotateurs de l'épaule.

15. Composition selon l'une des revendications 1 à 11 pour l'utilisation comme produit cosmétique.

16. Composition selon l'une des revendications 1 à 11 pour le traitement des lignes de froncement du visage, des rides du visage, des rides de la peau, des rides du contour de l'oeil, des sillons intersourcilliers, de la calvitie, de l'acné, de la transpiration excessive ou de la perte des cheveux.

## Claims

1. Composition comprising at least:
- one botulinum neurotoxin type A1, and
- one botulinum neurotoxin type A the amino acid sequence of which exhibits at least 5% difference from the amino acid sequence of the botulinum neurotoxin type A1.

2. Composition according to claim 1 **characterized in that** it comprises at least one botulinum neurotoxin type A the amino acid sequence of which exhibits at least 10% difference from the amino acid sequence of the botulinum neurotoxin type A1.

3. Composition according to one of the preceding claims **characterized in that** it comprises at least
- one botulinum neurotoxin type A1, and
- one botulinum neurotoxin type A2.

4. Composition according to one of the preceding claims **characterized in that** it comprises at least
- one botulinum neurotoxin type A1, and
- one botulinum neurotoxin type A2 isolated from the strain *Clostridium botulinum* NCTC9837.

5. Composition according to one of the preceding claims **characterized in that** it comprises at least one analgesic agent.

6. Composition according to one of the preceding claims **characterized in that** it comprises at least one polysaccharide.

7. Composition according to one of the preceding claims **characterized in that** it comprises at least one polysaccharide which is 2-hydroxy-ethyl starch.

8. Composition according to one of the preceding claims **characterized in that** it comprises at least one surfactant, chosen from the cationic, anionic or non-ionic surfactants.

9. Composition according to one of the preceding claims **characterized in that** it comprises at least one surfactant chosen from the non-ionic surfactants of the group of polysorbates.

10. Composition according to one of the preceding claims **characterized in that** it comprises at least one surfactant which is polysorbate 80.

11. Composition according to one of the preceding claims **characterized in that** it comprises at least one vasoconstrictor.

12. Composition according to one of claims 1 to 11, for use as a medicament.

13. Composition according to one of claims 1 to 11 for treatment of muscular disorders, neuromuscular disorders, neurological disorders, orthopaedic disorders, ophthalmological disorders, articular pathologies, endocrine disorders or urological disorders.

14. Composition according to one of claims 1 to 11 for treatment of torticollis, spasmodic torticollis, local disorders of spasticity of the upper and/or lower limbs, pain, muscle pain, pain due to muscle spasms, myofascial pain, post-operative pain, muscle spasms, hemifacial spasm, blepharospasm, strabismus, facial asymmetry, muscle dystonia, cerebral paralysis, headaches, migraine, fibromyalgia, myalgia, hyperhidrosis, bromhidrosis, coxarthrosis, arthrosis of the hip, epicondylitis of the elbow, arthritis, rheumatoid arthritis, dyskinesias, achalasia, Oddi's sphincter dysfunctions, pancreatitis, gout, anal fissures, constipation, anismus, spasms of the pyloric valve, spastic bladder, spasms of the bladder, urinary incontinence, urine retention, prostatic hyperplasia, endometriosis, psoriasis, rhinitis, allergic rhinitis, obesity, hyperlacrimation, bone fractures, tendon lacerations or rotator muscle cap pathology of the shoulder.

15. Composition according to one of claims 1 to 11 for use as a cosmetic product.

16. Composition according to one of claims 1 to 11 for treating facial frown lines, facial wrinkles, wrinkles of the skin, wrinkles of the contour of the eye, glabellar frown lines, baldness, acne, excessive perspiration or hair loss.

## Patentansprüche

1. Zusammensetzung, umfassend zumindest:
- ein Botulinumneurotoxin Typ A1, und
- ein Botulinumneurotoxin Typ A, dessen Aminosäuresequenz zumindest 5% Differenz zu der Aminosäuresequenz von Botulinumneurotoxin Typ A1 aufweist.

2. Zusammensetzung gemäss Anspruch 1, **dadurch gekennzeichnet, dass** sie zumindest ein Botulinumneurotoxin Typ A enthält, dessen Aminosäuresequenz zumindest 10% Differenz zu der Aminosäuresequenz von Botulinumneurotoxin Typ A1 aufweist.

3. Zusammensetzung gemäss einem der vorigen Ansprüche, **dadurch gekennzeichnet, dass** sie zumindest umfasst:
- ein Botulinumneurotoxin Typ A1, und
- ein Botulinumneurotoxin Typ A2.

4. Zusammensetzung gemäss einem der vorigen Ansprüche, **dadurch gekennzeichnet, dass** sie zumindest umfasst:
- ein Botulinumneurotoxin Typ A1, und
- ein Botulinumneurotoxin Type A2, das von dem Stamm *Clostridium botulinum* NCTC9837 isoliert wurde.

5. Zusammensetzung gemäss einem der vorigen Ansprüche, **dadurch gekennzeichnet, dass** sie zumindest ein schmerzstillendes Agens umfasst.

6. Zusammensetzung gemäss einem der vorigen Ansprüche, **dadurch gekennzeichnet, dass** sie zumindest ein Polysaccharid umfasst.

7. Zusammensetzung gemäss einem der vorigen Ansprüche, **dadurch gekennzeichnet, dass** sie zumindest ein Polysaccharid umfasst, welches 2-Hydroxyethyl-Stärke ist.

8. Zusammensetzung gemäss einem der vorigen Ansprüche, **dadurch gekennzeichnet, dass** sie zumindest ein Tensid umfasst, welches ausgewählt ist unter kationischen, anionischen oder nichtionischen Tensiden.

9. Zusammensetzung gemäss einem der vorigen Ansprüche, **dadurch gekennzeichnet, dass** sie zumindest ein nichtionisches Tensid der Gruppe der Polysorbate umfasst.

10. Zusammensetzung gemäss einem der vorigen Ansprüche, **dadurch gekennzeichnet, dass** sie zumindest ein Tensid umfasst, welches Polysorbat 80 ist.

11. Zusammensetzung gemäss einem der vorigen Ansprüche, **dadurch gekennzeichnet, dass** sie zumindest ein gefässverengendes Mittel umfasst.

12. Zusammensetzung gemäss einem der Ansprüche 1 bis 11 zur Verwendung als Medikament.

13. Zusammensetzung gemäss einem der Ansprüche 1 bis 11 zur Behandlung von Muskelerkrankungen, neuromuskulären Erkrankungen, neurologischen Erkrankungen, orthopädischen Erkrankungen, ophthalmologischen Erkrankungen, artikulären Pathologien, endokrinen Erkrankungen oder urologischen Erkrankungen.

14. Zusammensetzung gemäss einem der Ansprüche 1 bis 11 zur Behandlung von Torticollis, spasmodischem Torticollis, lokalen spastischen Erkrankungen der oberen und unteren Glieder, Schmerzen, Muskelschmerzen, Schmerzen, welche durch Muskelspasmen verursacht werden, myofaszialen Schmerzen, postoperativen Schmerzen, Muskelspasmen, hemifaszialen Spasmen, Blepharospasmus, Strabismus, faszialer Asymmetrie, Muskeldystonie, zerebraler Paralyse, Kopfschmerz, Migräne, Fibromyalgie, Myalgie, Hyperhidrose, Bromhidrose, Coxarthrose, Arthrose der Hüfte, Epicondylitis des Ellbogens, Arthritis, Rheumatoider Arthritis, Dyskinesien, Achalasie, Sphinkter von Oddi Dysfunktion, Pankreatitis, Gicht, Analfissuren, Konstipation, Anismus, Spasmen des Pylorussphinkters, spastischer Blase, Spasmen der Blase, urinärer Incontinenz, urinärer Retention, Prostatahyperplasie, Endometriose, Psoriasis, Rhinitis, allergischer Rhinitis, Dickleibigkeit, Hyperlakrimation, Knochenbrüchen, Sehnenrissen oder krankhaften Veränderungen der Rotatorenmanschette der Schulter.

15. Zusammensetzung gemäss einem der Ansprüche 1 bis 11 zur Verwendung als kosmetisches Produkt.

16. Zusammensetzung gemäss einem der Ansprüche 1 bis 11 zur Behandlung von Gesichtsrunzeln, Gesichtsfalten, Hautfalten, Falten der Augenkontur, Glabellafalten, Kahlheit, Akne, übermässigem Schwitzen oder Haarverlust.
